# EUROPÄISCHE PATENTSCHRIFT

(11) **EP 2 467 751 B1**
(45) Veröffentlichungstag und Bekanntmachung des Hinweises auf die Patenterteilung: **31.10.2018**
(21) Anmeldenummer: 10722933.8
(22) Anmeldetag: 08.05.2010
(51) Int. Cl.: G02C 13/00, G02C 7/02, A61B 5/11

(54) **METHODE UND VORRICHTUNG ZUR ERMITTLUNG DER HABITUELLEN KOPFHALTUNG**
METHOD AND APPARATUS FOR DETERMINING THE HABITUAL HEAD POSTURE
PROCÉDÉ ET APPAREIL POUR DÉTERMINER LA POSTURE HABITUELLE DE LA TÊTE

(30) Priorität: 17.06.2009 DE 102009025215
(43) Veröffentlichungstag der Anmeldung: 27.06.2012
(60) Teilanmeldung: 18188534.4
(73) Patentinhaber: Carl Zeiss Vision International GmbH, 73430 Aalen (DE)
(72) Erfinder: KUBITZA, Matthias, 73431 Aalen (DE); GAMPERLING, Michael, 89340 Leipheim (DE); CABEZA-GUILLÉN, Jesús Miguel, 73434 Aalen (DE)
(74) Vertreter: Carl Zeiss AG - Patentabteilung
(86) Internationale Anmeldenummer: PCT/EP2010/002821
(87) Internationale Veröffentlichungsnummer: WO 2010/145736

(56) Entgegenhaltungen:
- EP-A1- 1 637 067
- EP-A2- 0 898 930
- WO-A1-2006/029875
- WO-A2-2007/045694
- DE-A1-102004 063 160
- DE-A1-102008 018 198
- FR-A1- 2 896 682
- US-A1- 2003 143 391
- US-A1- 2009 207 375

## Beschreibung

Die Erfindung betrifft ein Verfahren zur Zentrierdatenermittlung nach dem Oberbegriff des Patentanspruchs 1 sowie eine Vorrichtung zur Zentrierdatenermittlung nach dem Oberbegriff des Patentanspruchs 11. Schließlich betrifft die Erfindung ein Computerprogramm zur Durchführung des vorstehend angegebenen Verfahrens.

Wenn Augenoptiker zur optischen Anpassung einer Brille die Zentrierdaten, also alle notwendigen Angaben zur korrekten Zentrierung von Brillengläsern in einer Brillenfassung für den individuellen Anwendungszweck des Kunden oder Probanden, ermittelt, so kann wie folgt verfahren werden:
Der Kunde wird gebeten eine möglichst natürliche Kopf- und Körperhaltung einzunehmen. Die Festlegung der Zentrierwerte erfolgt dann durch Markierung der Durchblickpunkte in der Scheibenebene der Brillenfassung mittels Stift oder mittels Videoaufnahme und anschließender, manueller (wie z.B. bei der von der Anmelderin unter der Marke "Video Infral" vertriebenen Videozentriereinrichtung) bzw. automatisierter (wie z.B. bei der von der Anmelderin unter der Marke "RV Terminal" vertriebenen Videozentriereinrichtung) Zentrierdatenermittlung.

Zur Ermittlung des Vorneigungswinkels ist ein Hilfsmittel Namens "Y-Stick" bekannt, welches in einem Zeitpunkt den Vorneigungswinkel der Brillenfassung "einfriert", damit dieser anschließend abgelesen werden kann. Der so erfasste Vorneigungswinkel wird in einem nachfolgenden Schritt dazu benutzt, den Kunden in gleicher Position vor einem Zentriersystem zu positionieren.

Weiterhin ist aus der EP 1 591 064 A1 ein Messgerät zur Bestimmung der Kopfneigung bekannt, welches von der Anmelderin der EP 1 591 064 unter der Bezeichnung "Ysis" vertrieben wird. Dieses Messgerät wird an der Brillenfassung befestigt. Dieser Vorschlag zielt darauf ab, die "normale" Kopfhaltung zu einem Zeitpunkt jeweils für Nah- und Fernblickrichtung festzuhalten. Aus den Kopfneigungen, die beim Blick in die Ferne sowie beim Lesen eingenommen werden, wird der Unterschied der Kopfneigung zwischen Fern- und Nahsehaufgaben ermittelt. Daraus werden dann die zugehörigen Durchblickpunkte durch das Brillenglas bestimmt.

Sämtliche vorstehenden Methoden sind statisch, d.h. zur Ermittlung einer habituellen Kopfhaltung wird eine "Momentaufnahme" der jeweiligen Situation herangezogen.

Die WO 01/62139 A1 offenbart, das Verhalten eines Brillenträgers hinsichtlich seiner Blickgewohnheiten zu klassifizieren. Es wird die typische Kopf- und Augenbewegung bei verschiedenen Sehaufgaben bestimmt. Davon abhängig, wird das Blickverhalten des Probanden kategorisiert nach "Beweglichkeit" der Augen und des Kopfes ("head/eye mover"). Die Ergebnisse der Analyse werden dabei als Grundlage für die Auswahl eines geeigneten Brillenglases verwendet.

Aus der EP 1 747 750 A1 ist es bekannt, die häufigsten Blickrichtungen relativ zum Kopf festzustellen. Zu diesem Zweck werden Kopfhaltung und Blickrichtung gleichzeitig detektiert. Aus den ermittelten relativen Blickrichtungen wird die relative "Netto"-Blickrichtung und der daraus resultierende Durchblickpunkt durch ein Brillenglas bestimmt.

Die DE 10 2004 063 160 A1 beschreibt ein Verfahren und eine Einrichtung zum Anpassen einer Brille, insbesondere zur Erfassung der Habitus-Kopfhaltung eines Probanden, dessen bzw. deren Messdaten Eingang finden bei der Video-Zentrierdaten-Bestimmung. Bei dem Verfahren werden vom Probanden mehrere Video-Fotos zur Ermittlung der Kopfhaltungswinkel eines Probanden in verschiedenen Kopfhaltungspositionen gefertigt, die Kopfhaltungswinkel erfasst und mit dem Vorneigungswinkel der Brillenfassung sowie dem Hornhautscheitelabstand abgeglichen. Die entsprechenden Video-Fotos werden in einem Moment aufgenommen, wenn nach Meinung des Bedienenden der Proband seine natürliche Habitus-Kopfhaltung eingenommen hat.

Die FR 2 896 682 A2 beschreibt ein Gerät zur Bestimmung des Winkels zwischen der Blickrichtung eines Probanden in der Nähe und der Blickrichtung des Probanden in die Ferne. Es erfolgt eine Aufnahme des Probanden mit einer Kamera oder einem Fotoapparat wenn er liest und wenn er in die Ferne blickt. Ob der Proband eine habituelle Kopfhaltung einnimmt wird nicht überprüft.

Die nachveröffentlichte DE 10 2008 018 198 A1 beschreibt die benutzerabhängige Anpassung von Brillengläsern an ein Brillengestell. Dazu wird vorbereitend zunächst eine Kopfneigung eines Benutzers während einer Kalibrier-Sehsequenz gemessen. Während der Kalibrier-Sehsequenz wird eine Vielzahl von Kopfneigungswerten gespeichert. Aus den gespeicherten Kopfneigungswerten wird eine Nullblick-Kopfneigung ermittelt. Nach dem Ermitteln der Nullblick-Kopfneigung wird die Augenposition des Benutzers relativ zum Brillengestell bei voreingestellter Nullblick-Kopfneigung des Benutzers vermessen. Zudem werden Vorrichtungen einerseits zur Durchführung des Vorbereitungsverfahrens und andererseits zur Durchführung des Anpassungsverfahrens vorgestellt.

Die US 2003/143391 A1 beschreibt die Erfassung der Kopfneigung eines Probanden beim Blick in die Nähe und in die Ferne mit Hilfe einer Videokamera. Der Proband löst die Erfassung mit Hilfe einer Taste aus. Ob der Proband gerade habituelle Kopfneigung innehat ist mit dieser Methode nicht sichergestellt.

Die EP 0 898 930 A2 beschreibt ein Verfahren zur Bestimmung von Blickwinkeln bei unterschiedlichen Kopfpositionen und zugleich unterschiedlicher Blickrichtung. Mit einer Videokamera werden am Kopf der Probanden angebrachte Lichtquellen erfasst während er auf einem Bildschirm oder dergleichen eingeblendete Hinweise fixiert.

Die WO 2007/045694 A2 beschreibt die Aufzeichnung und Ausweitung von Kopfbewegungen eines Probanden, wenn sein Blick einem vorgegebenen Weg folgt.

Die WO 2006/029875 A1 die als nächstligender Stend der Technik angesehen wird beschreibt einen Messbügel, welcher die Bestimmung einer Kopfdrehung zwischen einer idealen, zu einer Seitenkamera senkrechten Ausrichtung des Kopfs und einer tatsächlichen habituellen Ausrichtung ermöglicht. Da der von der Seitenkamera unmittelbar erfasste Hornhautscheitelabstand im Falle einer habituellen Kopfausrichtung nicht dem tatsächlichen Hornhautscheitelabstand entspricht, erfolgt eine Korrektur auf Basis der mit dem Messbügel bestimmten Kopfdrehung.

Eine Zentriermessung, also eine Messung, bei der alle notwendigen Angaben zur korrekten Zentrierung von Brillengläsern in einer Brillenfassung für den individuellen Anwendungszweck des Kunden oder Probanden, ermittelt werden, stellt für den Kunden des Optikers eine erzwungene Situation dar. Zwar ist aus der Zentriermessung bekannt, wie der Kunde während einer Aufnahme den Kopf hielt, diese Kopfhaltung entspricht allerdings nicht zwangsläufig seiner "normalen", d.h. häufigsten Kopfhaltung in tatsächlich ungezwungenen Situationen.

Dies ändert sich auch nicht, wenn das in der DE 10 2004 063 160 A1 beschriebene Verfahren zum Einsatz kommt. Darüber hinaus birgt dieses Verfahren die Gefahr, dass gar nicht die vermeintlich natürliche Kopfhaltung aufgenommen wird, sondern eine andere, weil der Proband innerhalb der Reaktionszeit der Bedienenden bereits eine andere Kopfhaltung eingenommen hat.

Die Aufgabe der Erfindung besteht nunmehr in der Bereitstellung eines präzisierten Verfahrens zur Zentrierdatenermittlung und einer entsprechenden Vorrichtung, welche geeignet ist, das erfindungsgemäße Verfahren auszuführen.

Die Aufgabe wird durch ein gattungsgemäßes Verfahren zur Zentrierdatenermittlung mit den Merkmalen des kennzeichnenden Teils des Patentanspruchs 1 bzw. eine entsprechende gattungsgemäße Vorrichtung mit den Merkmalen des kennzeichnenden Teils des Patentanspruchs 11 gelöst.

Vorteilhafte Ausführungen und Weiterbildungen der Erfindung sind Gegenstand der Unteransprüche.

Die Erfindung umfasst ein Verfahren zur Zentrierdatenermittlung gemäß des unabhängigen Anspruchs 1.

In entsprechender Weise ist Gegenstand der Erfindung auch eine Vorrichtung zur Zentrierdatenermittlung gemäß des Anspruchs 11.

Die oben angegebene Aufgabe wird durch ein derartiges Verfahren bzw. die entsprechende Vorrichtung vollumfänglich gelöst.

Ein erfindungsgemäß verwendbares Verfahren zur Ermittlung der habituellen Kopfhaltung eines Probanden umfasst die Verfahrensrensschritte gemäß des Ansprüchs 3.

Die entsprechende erfindungsgemäß verwendbare Vorrichtung zur Ermittlung der habituellen Kopfhaltung des Probanden umfasst in korrespondierender Weise die Merkmale gemäß des Anspruchs 14.

Durch das vorstehend skizzierte Verfahren bzw. die insbesondere Durchführung dieses Verfahrens ausgebildete entsprechende Vorrichtung wird vermieden, dass die habituelle Kopfhaltung des Probanden irrtümlich als eine zufällig zu einem Zeitpunkt aufgenommene bzw. bestimmte Kopfhaltung angenommen wird, die gerade nicht seiner gewöhnlichen Kopfhaltung entspricht.

Die Annahme der habituellen Kopfhaltung als die aus einer über eine gewisse Zeit hinweg aufgezeichneten Kopfhaltung ermittelte bevorzugte Kopfhaltung ermöglicht es z.B. auch, die bei der Zentriermessung (z.B. mit Hilfe der unter den Bezeichnungen Relaxed Vision Terminal und Video Infral von der Anmelderin vertriebenen Videozentriergeräten) gemessene Kopfhaltung zu korrigieren, so dass die Zentrierdaten auf Basis der "normalen" und nicht der zufälligen Kopfhaltung bestimmt werden.

Bei dem erfindungsgemäß verwendbaren Verfahren zur Ermittlung der habituellen Kopfhaltung eines Probanden kann die Zeitspanne, innerhalb der die Kopfhaltung aufgezeichnet wird, vorgegeben werden. So kann zum Beispiel die Kopfhaltung nur während der Zeit aufgezeichnet werden, während der sich auch verlässliche Daten zur habituellen Haltung des Kopfes des Probanden entnehmen lassen. Die Aufzeichnung erzwungener und/oder unnatürlicher Kopfhaltungen kann vermieden werden. Darüber hinaus lässt sich die Zeitspanne individuell so festlegen, dass das aufgenommene Datenmaterial eine hinreichend verlässliche Aussage über die tatsächliche habituelle Kopfhaltung zulässt. Günstig ist es z.B., wenn aus der Aufzeichnung nur solche Zeitabschnitte zur Ermittlung der habituellen Kopfhaltung ausgewählt werden, innerhalb derer der Proband auch eine Kopf- und/oder Körperhaltung eingenommen hat, die ergonomischen Grundsätzen entspricht.

Als bevorzugte Kopfhaltung ist die Kopfhaltung zu verstehen, in der der Proband seinen Kopf bei einer vorgegebenen Sehaufgabe mit einer gewissen Wahrscheinlichkeit hält. Typische Werte für diese Wahrscheinlichkeit sind z.B. über 30 %, über 50 %, über 80 % oder gar über 90 % für die bestimmte Sehaufgabe. Eine derartige Analyse lässt sich mit üblichen mathematischen/statistischen Methoden durchführen. Geeignete Software Algorithmen können bei Bedarf automatisch oder mit Unterstützung durch den Benutzer Extremwerte (Ausreißer) aus der aufgezeichneten Datenmenge der habituellen Kopfhaltung eliminieren, bevor eine Bestimmung einer typischen Kopfhaltung erfolgt. Es erfolgt dann z.B. ein Auswahl aus der Menge dieser wahrscheinlichsten Kopfhaltungen.

Als bevorzugte Kopfhaltung kann auch die aufgezeichnete Haltung des Kopfes gewählt werden, in der der Proband den Kopf mit der größten Wahrscheinlichkeit hält.

Obwohl es grundsätzlich möglich ist, dem Probanden freizustellen, in welcher Körperhaltung und/oder was er bei der Aufzeichnung seiner Kopfhaltung betrachtet, ist es vielfach günstig, diesem bestimmte Sehaufgaben aufzuerlegen. Insbesondere wenn die Kopfhaltung Eingang in die Brillenglasberechnung oder die Zentrierung eines Brillenglases findet, ist es sinnvoll, besondere Aufmerksamkeit den Blickrichtungen für die Nähe und/oder die Ferne zu widmen. Die Erfindung sieht z.B. vor, als bevorzugte Kopfhaltung eine der aufgezeichneten Haltungen zu wählen, in der der Proband den Kopf beim Blick in die Nähe und/oder beim Blick in die Ferne hält.

Weiterhin ist es sinnvoll, die Körperhaltung des Probanden zu beachten. Beim Blick in die Ferne wird eine Person eher stehen, wohingegen eine Person beim Blick in die Nähe, wie z.B. beim Lesen, bevorzugt eine sitzende Position einnimmt. Die Erfindung sieht daher vor, als bevorzugte Kopfhaltung eine der aufgezeichneten Haltungen zu wählen, während der der Proband steht und/oder sitzt.

Die habituelle Kopfhaltung hängt in besonderem Maß von der Tätigkeit des Probanden und den visuellen Eindrücken, die der Proband wahrnimmt ab. Es ist daher weiter günstig, dem Probanden konkrete Alltagssituationen und/oder alltägliche Sehaufgaben darzubieten unter denen seine habituelle Kopfhaltung bestimmt werden soll. Der hiermit verbundene Vorteil liegt darin, dass der Proband/Kunde von der eigentlichen Messsituation abgelenkt ist und damit keine unnatürliche Kopfhaltung einnimmt. Die Sehaufgaben können darin bestehen, dass der Proband/Kunde sich z.B. im Laden des Optikers, der die habituelle Kopfhaltung aufzeichnet frei bewegt und z.B. die Auslagen betrachtet oder dass er z.B. Verkehrssituationen verfolgt, die über ein elektronisches Medium, wie ein Display, einen Beamer usw., dargestellt werden, wie dies z.B. in der US 2007/0229761A1 beschrieben ist oder dass er gebeten wird ein Schriftstück durchzulesen. Die Sehaufgaben werden z.B. so gewählt, dass eine typische Kopfhaltung im Stehen und/oder im Sitzen bestimmt werden kann. Bei der Zentrierdatenermittlung für eine Lesebrille wird z.B. die habituelle Kopfhaltung zu berücksichtigen sein, während der der Proband sitzt und liest.

Konkret können dem Probanden/Kunden zur dynamischen Ermittlung der habituellen Kopfhaltung über ein Zeitintervall Alltagssituationen bzw. alltägliche Sehaufgaben im Nah- und/oder Fernbereich dargeboten werden. In dieser Zeit werden die Kopfhaltung, ggf. in Kombination mit der zugehörigen Körperhaltung und ggf. auch die (relative) Position wie z.B. die Neigung der Brille bzw. der Brillengläser, die der Proband trägt, fortlaufend gemessen. Die Messung erfolgt so, dass der Proband/Kunde davon nicht beeinträchtigt wird. Im Anschluss an die Messung kann die jeweils häufigste Kopfhaltung, soweit mit aufgezeichnet auch die zugehörige Körperhaltung- und ggf. auch die Position, insbesondere Neigung der Brille bzw. der Brillengläser bestimmt und später bei der Ermittlung der Zentrierdaten berücksichtigt werden. Insbesondere können die Durchblickpunkte bei Nah- und/oder Fernsehaufgaben, der sogenannte Inset und/oder die Korridorlänge bestimmt werden. Konkret kann z.B. aus der Kopfhaltung beim Sehen im Nah- und Fernbereich bei bekannter Position des Sehziels der Durchblickpunkt durch das Brillenglas bestimmt werden. Sind die Durchblickpunkte bekannt, kann damit auch die Größe des Insets bestimmt werden, sowie die Länge des Korridors des Nahteils. Ein eventueller Unterschied zwischen Kopfrichtung und Blickrichtung kann bei der Bestimmung der Durchblickpunkte und/oder des Insets mit berücksichtigt werden.

Die Kopfhaltung des Probanden kann auf unterschiedlichste Art und Weise ermittelt werden. So kann z.B. die Aufzeichnung der Kopfhaltung mittels eines am Kopf des Probanden angebrachten Positionsaufzeichnungsgeräts durchgeführt werden. Dieses Positionsaufzeichnungsgerät kann mittelbar also z.B. am Brillenfassungsbügel oder unmittelbar am Kopf des Klienten befestigt sein. Dieses Positionsaufzeichnungsgerät kann nach dem Prinzip eines Datenloggers mit anschließendem Datentransfer über eine Basisstation funktionieren oder der Datentransfer kann zeitgleich über eine drahtlose Verbindung, wie WPAN (Wireless Personal Area Network), nämlich z.B. via Bluetooth, oder WLAN (Wireless Local Area Network) online während des gesamten Messintervalls erfolgen. Das Positionsaufzeichnungsgerät kann entweder absolute Lageinformationen messen oder aber Änderungen in der Lage. Im zweiten Fall muss der Messwertaufnehmer vor und/oder nach dem Messvorgang in eine Referenzlage gebracht werden, deren Ausrichtung bekannt ist. Als Referenzlage kann z.B. eine horizontale Tischfläche oder die Auflage einer speziell dafür vorgesehenen Halterung dienen.

Die Aufzeichnung der Kopfhaltung kann zusätzlich oder alternativ mittels eines Bildaufzeichnungsgeräts durchgeführt werden. Ein derartiges Bildaufzeichnungsgerät kann z.B. ein Video- oder Digitalkamerasystem mit angeschlossener automatisierter Bildauswertung sein. Die Bilddaten können entweder als zweidimensionale (2D) Daten direkt oder nach Erstellung eines dreidimensionalen (3D) Modells als 3D-Daten weiterverarbeitet werden. Aus diesen Daten können z.B. auch typische Parameter wie Vorneigung, Seitenneigung, usw., der Brille bestimmt werden.

Bei dem erfindungsgemäßen Verfahren zur Zentrierdatenermittlung wird die bestimmte tatsächliche momentane Kopfhaltung mit Hilfe der bestimmten habituellen Kopfhaltung derart korrigiert, dass die Zentrierdaten auf Basis der habituellen und nicht der zufälligen Kopfhaltung bestimmt werden. Die Korrektur erfolgt indem die Abweichung der momentanen Kopfhaltung von der habituellen Kopfhaltung ermittelt wird und anhand dieser Abweichung eine Umrechnung/Korrektur der Zentrierwerte erfolgt.

Die ermittelte habituelle Kopfhaltung des Probanden/Kunden und der ggf. ebenfalls ermittelte Vorneigungswinkel der Brille können auch zur erzwungenen Ausrichtung des Kunden für die konventionelle Zentrierdatenermittlung dienen, indem bei Erreichen des angestrebten Vomeigungswinkels der Brille eine automatische Auslösung der bildgebenden Einheiten eingeleitet wird bzw. ein Signal dem Operator gegeben wird, dass nun die Zentrierdatenermittlung erfolgen kann.

Die Erfindung sieht insbesondere auch vor, dass die vorstehend beschriebenen Verfahren zur Ermittlung der habituellen Kopfhaltung sowie zur Zentrierdatenermittlung in Form von in einem Computer ausführbaren Computerprogrammen mit Programmcodes vorliegen. Die Computerprogramme können auf einem maschinenlesbaren Datenträger gespeichert sein.

Der Vollständigkeit halber wird darauf hingewiesen, dass die o.a. Aufzeichnungseinrichtung zur Aufzeichnung der Kopfhaltung über eine Zeitspanne ein am Kopf des Probanden anbringbares Positionsaufzeichnungsgerät und/oder ein Bildaufzeichnungsgerät umfassen kann. Positionsaufzeichnungsgerät und/oder Bildaufzeichnungsgerät können z.B. in der vorstehend beschriebenen Weise ausgebildet sein. Die Ermittlungseinrichtung zur Ermittlung einer bevorzugten Kopfhaltung aus der Aufzeichnung kann einen Computer umfassen.

Die Kopfhaltungsbestimmungseinrichtung zur Bestimmung der tatsächlichen momentanen Kopfhaltung eines Probanden kann ein Positionsaufzeichnungsgerät und/oder ein Bildaufzeichnungsgerät, insbesondere der vorstehend beschriebenen Art, umfassen.

Die Ermittlungseinrichtung zur Ermittlung der Anordnung einer Brillenfassung relativ zur bestimmten tatsächlichen momentanen Kopfhaltung kann ein Bildaufzeichnungsgerät, insbesondere eine Video- oder Digitalkamera, und/oder einen Computer umfassen.

Die Erfindung wird nunmehr anhand der Zeichnung näher beschrieben. Es zeigen:
- Figur 1: eine schematische Darstellung einer erfindungsgemäßen Vorrichtung zur Zentrierdatenermittlung mit erfindungsgemäßer Vorrichtung zur Ermittlung der habituellen Kopfhaltung eines Probanden;
- Figur 2: Aufnahmen des Gesichts eines Kunden mit angepasster Fassung in Nullblickrichtung
a. von vorn
b. von der Seite
c. von vorn mit Zentrierdaten, nämlich x, y
d. von der Seite mit Zentrierdaten, nämlich Hornhautscheitelabstand und Fassungsvorneigung

Es wird angenommen, dass ein Kunde ein Optikergeschäft zum Zwecke des Erwerbs einer neuen Brille aufsucht. Zunächst wird er hinsichtlich der für ihn in Frage kommenden Brillengläser und Fassungen beraten. Nach erfolgter Auswahl der Fassung erfolgt die eigentliche Zentrierdatenermittlung mit Hilfe einer erfindungsgemäßen Vorrichtung.

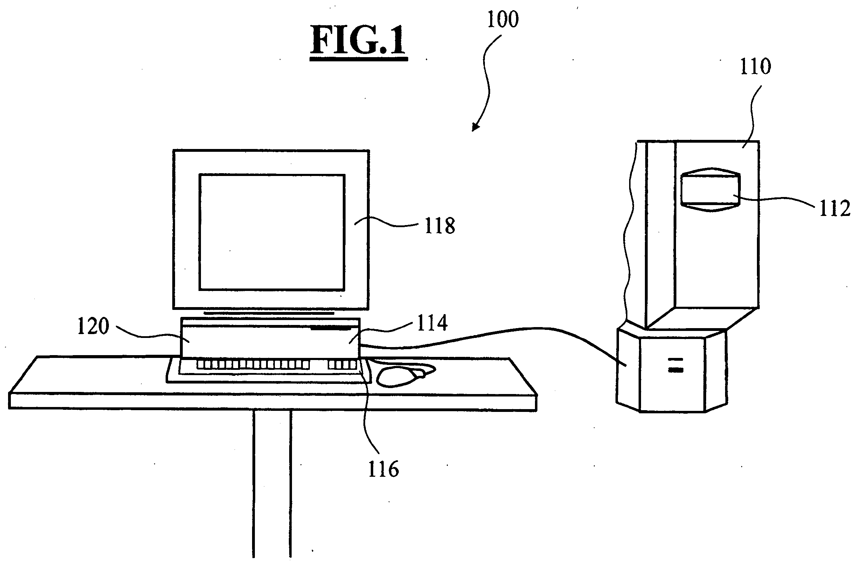

Die Figur 1 zeigt eine erfindungsgemäße Vorrichtung zur Zentrierdatenermittlung 100. Diese Vorrichtung zur Zentrierdatenermittlung 100 umfasst ein Videozentriergerät 110 mit Videokamera 112 und Computer 114 mit Tastatur 116, Bildschirm 118 und Speichermedium 120. Es handelt sich um ein kalibriertes System, also ein System zur Aufnahme und Bestimmung von Abmessungen.

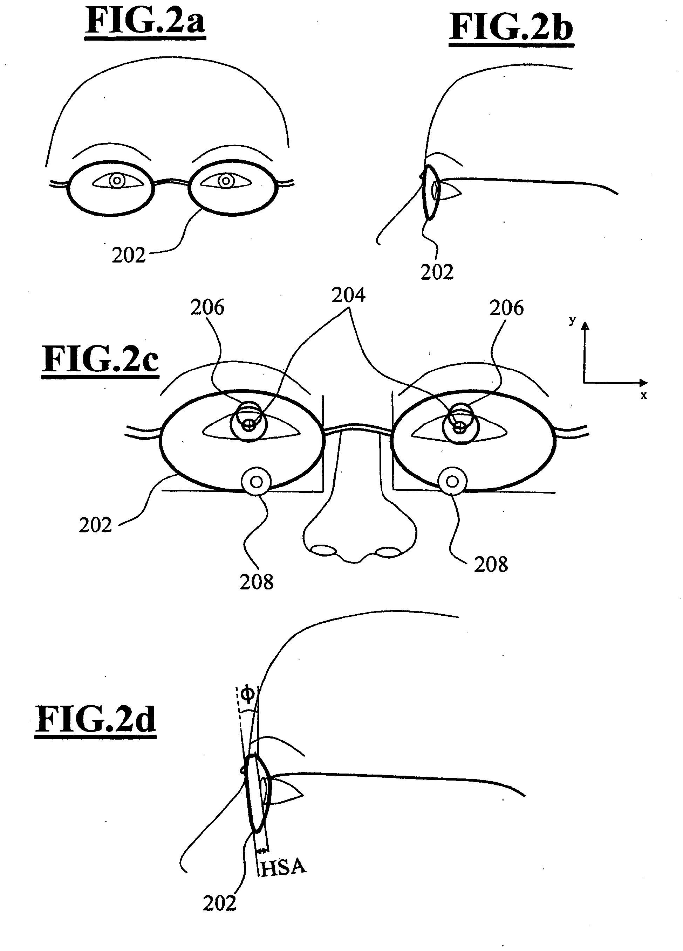

Das Gesicht des Kunden wird mit angepasster Fassung 202 in Nullblickrichtung von vorn und von der Seite mit der Videokamera 112 aufgenommen, wie dies in den Figuren 2a) und b) dargestellt ist.

Mit der auf den Bildschirm 118 dargestellten Frontaufnahme (Figur 2c) wird zunächst die Fassungsform 202 erfasst. Weiterhin werden die Pupillenmitten 204, z.B. im Koinzidenzverfahren, relativ zur Fassungsform 202 ermittelt. Mit der Seitenaufnahme werden der Hornhautscheitelabstand HSA und die Fassungsvorneigung ϕ zur Erfüllung der Augendrehpunktforderung ermittelt, wie dies in Figur 2d) dargestellt ist.

Erfindungsgemäß ist nunmehr vorgesehen, die habituelle Kopfhaltung und die Neigung der Brille z.B. mit Hilfe der kalibrierten Videokamera über einen längeren Zeitraum aufzuzeichnen und hieraus eine habituelle Kopfhaltung und eine bevorzugte Neigung der Brille zu ermitteln.

Die somit ermittelte Kopfhaltung und der damit zusammenhängende Vorneigungswinkel der Brille sowie andere Messdaten wie z.B. die Anordnung der Brille in der Fassungs- oder Scheibenebene in x, y dienen zur Umrechnung der mittels des Videozentriergeräts 110 konventionell ermittelten Zentrierdaten (z.B. Fernbezugspunkt 206, Nahmesskreis 208), falls bei deren Bestimmung der Kunde eine von der normalen Kopfhaltung abweichende Kopfhaltung eingenommen hatte (vgl. Figur 2c).

### Beschreibung

### Methode und Vorrichtung zur Ermittlung der habituellen Kopfhaltung

Die Erfindung betrifft ein Verfahren zur Zentrierdatenermittlung nach dem Oberbegriff des Patentanspruchs 1 sowie eine Vorrichtung zur Zentrierdatenermittlung nach dem Oberbegriff des Patentanspruchs 9. Schließlich betrifft die Erfindung ein Computerprogramm zur Durchführung des vorstehend angegebenen Verfahrens.

Wenn Augenoptiker zur optischen Anpassung einer Brille die Zentrierdaten, also alle notwendigen Angaben zur korrekten Zentrierung von Brillengläsern in einer Brillenfassung für den individuellen Anwendungszweck des Kunden oder Probanden, ermittelt, so kann wie folgt verfahren werden:
Der Kunde wird gebeten eine möglichst natürliche Kopf- und Körperhaltung einzunehmen. Die Festlegung der Zentrierwerte erfolgt dann durch Markierung der Durchblickpunkte in der Scheibenebene der Brillenfassung mittels Stift oder mittels Videoaufnahme und anschließender manueller (wie z.B. bei der von der Anmelderin unter der Marke "Video Infral" vertriebenen Videozentriereinrichtung) bzw. automatisierter (wie z.B. bei der von der Anmelderin unter der Marke "RV Terminal" vertriebenen Videozentriereinrichtung) Zentrierdatenermittlung.

Zur Ermittlung des Vorneigungswinkels ist ein Hilfsmittel Namens "Y-Stick" bekannt, welches in einem Zeitpunkt den Vorneigungswinkel der Brillenfassung "einfriert", damit dieser anschließend abgelesen werden kann. Der so erfasste Vorneigungswinkel wird in einem nachfolgenden Schritt dazu benutzt, den Kunden in gleicher Position vor einem Zentriersystem zu positionieren.

Weiterhin ist aus der EP 1 591 064 A1 ein Messgerät zur Bestimmung der Kopfneigung bekannt, welches von der Anmelderin der EP 1 591 064 unter der Bezeichnung "Ysis" vertrieben wird. Dieses Messgerät wird an der Brillenfassung befestigt. Dieser Vorschlag zielt darauf ab, die "normale" Kopfhaltung zu einem Zeitpunkt jeweils für Nah- und Fernblickrichtung festzuhalten. Aus den Kopfneigungen, die beim Blick in die Ferne sowie beim Lesen eingenommen werden, wird der Unterschied der Kopfneigung zwischen Fern- und Nahsehaufgaben ermittelt. Daraus werden dann die zugehörigen Durchblickpunkte durch das Brillenglas bestimmt.

Sämtliche vorstehenden Methoden sind statisch, d.h. zur Ermittlung einer habituellen Kopfhaltung wird eine "Momentaufnahme" der jeweiligen Situation herangezogen.

Die WO 01/62139 A1 offenbart, das Verhalten eines Brillenträgers hinsichtlich seiner Blickgewohnheiten zu klassifizieren. Es wird die typische Kopf- und Augenbewegung bei verschiedenen Sehaufgaben bestimmt. Davon abhängig, wird das Blickverhalten des Probanden kategorisiert nach "Beweglichkeit" der Augen und des Kopfes ("head/eye mover"). Die Ergebnisse der Analyse werden dabei als Grundlage für die Auswahl eines geeigneten Brillenglases verwendet.

Aus der EP 1 747 750 A1 ist es bekannt, die häufigsten Blickrichtungen relativ zum Kopf festzustellen. Zu diesem Zweck werden Kopfhaltung und Blickrichtung gleichzeitig detektiert. Aus den ermittelten relativen Blickrichtungen wird die relative "Netto"-Blickrichtung und der daraus resultierende Durchblickpunkt durch ein Brillenglas bestimmt.

Die DE 10 2004 063 160 A1 beschreibt ein Verfahren und eine Einrichtung zum Anpassen einer Brille, insbesondere zur Erfassung der Habitus-Kopfhaltung eines Probanden, dessen bzw. deren Messdaten Eingang finden bei der Video-Zentrierdaten-Bestimmung. Bei dem Verfahren werden vom Probanden mehrere Video-Fotos zur Ermittlung der Kopfhaltungswinkel eines Probanden in verschiedenen Kopfhaltungspositionen gefertigt, die Kopfhaltungswinkel erfasst und mit dem Vorneigungswinkel der Brillenfassung sowie dem Hornhautscheitelabstand abgeglichen. Die entsprechenden Video-Fotos werden in einem Moment aufgenommen, wenn nach Meinung des Bedienenden der Proband seine natürliche Habitus-Kopfhaltung eingenommen hat.

Die FR 2 896 682 A2 beschreibt ein Gerät zur Bestimmung des Winkels zwischen der Blickrichtung eines Probanden in der Nähe und der Blickrichtung des Probanden in die Ferne. Es erfolgt eine Aufnahme des Probanden mit einer Kamera oder einem Fotoapparat wenn er liest und wenn er in die Ferne blickt. Ob der Proband eine habituelle Kopfhaltung einnimmt wird nicht überprüft.

Die nach veröffentlichte DE 10 2008 018 198 A1 beschreibt die benutzerabhängige Anpassung von Brillengläsern an ein Brillengestell. Dazu wird vorbereitend zunächst eine Kopfneigung eines Benutzers während einer Kalibrier-Sehsequenz gemessen. Während der Kalibrier-Sehsequenz wird eine Vielzahl von Kopfneigungswerten gespeichert. Aus den gespeicherten Kopfneigungswerten wird eine Nullblick-Kopfneigung ermittelt. Nach dem Ermitteln der Nullblick-Kopfneigung wird die Augenposition des Benutzers relativ zum Brillengestell bei voreingestellter Nullblick-Kopfneigung des Benutzers vermessen. Zudem werden Vorrichtungen einerseits zur Durchführung des Vorbereitungsverfahrens und andererseits zur Durchführung des Anpassungsverfahrens vorgestellt.

Die US 2003/143391 A1 beschreibt die Erfassung der Kopfneigung eines Probanden beim Blick in die Nähe und in die Ferne mit Hilfe einer Videokamera. Der Proband löst die Erfassung mit Hilfe einer Taste aus. Ob der Proband gerade habituelle Kopfneigung innehat ist mit dieser Methode nicht sichergestellt.

Die EP 0 898 930 A2 beschreibt ein Verfahren zur Bestimmung von Blickwinkeln bei unterschiedlichen Kopfpositionen und zugleich unterschiedlicher Blickrichtung. Mit einer Videokamera werden am Kopf der Probanden angebrachte Lichtquellen erfasst während er auf einem Bildschirm oder dergleichen eingeblendete Hinweise fixiert.

Die WO 2007/045694 A2 beschreibt die Aufzeichnung und Auswertung von Kopfbewegungen eines Probanden, wenn sein Blick einem vorgegebenen Weg folgt.

Die WO 2006/029875 A1 die als nächstliegender Stand der Technik angeschen wird beschreibt einen Messbügel, welcher die Bestimmung einer Kopfdrehung zwischen einer idealen, zu einer Seitenkamera senkrechten Ausrichtung des Kopfs und einer tatsächlichen habituellen Ausrichtung ermöglicht. Da der von der Seitenkamera unmittelbar erfasste Hornhautscheitelabstand im Falle einer habituellen Kopfausrichtung nicht dem tatsächlichen Hornhautscheitelabstand entspricht, erfolgt eine Korrektur auf Basis der mit dem Messbügel bestimmten Kopfdrehung.

Eine Zentriermessung, also eine Messung, bei der alle notwendigen Angaben zur korrekten Zentrierung von Brillengläsern in einer Brillenfassung für den individuellen Anwendungszweck des Kunden oder Probanden, ermittelt werden, stellt für den Kunden des Optikers eine erzwungene Situation dar. Zwar ist aus der Zentriermessung bekannt, wie der Kunde während einer Aufnahme den Kopf hielt, diese Kopfhaltung entspricht allerdings nicht zwangsläufig seiner "normalen", d.h. häufigsten Kopfhaltung in tatsächlich ungezwungenen Situationen.

Dies ändert sich auch nicht, wenn das in der DE 10 2004 063 160 A1 beschriebene Verfahren zum Einsatz kommt. Darüber hinaus birgt dieses Verfahren die Gefahr, dass gar nicht die vermeintlich natürliche Kopfhaltung aufgenommen wird, sondern eine andere, weil der Proband innerhalb der Reaktionszeit der Bedienenden bereits eine andere Kopfhaltung eingenommen hat.

Die Aufgabe der Erfindung besteht nunmehr in der Bereitstellung eines präzisierten Verfahrens zur Zentrierdatenermittlung und einer entsprechenden Vorrichtung, welche geeignet ist, das erfindungsgemäße Verfahren auszuführen.

Die Aufgabe wird durch ein gattungsgemäßes Verfahren zur Zentrierdatenermittlung mit den Merkmalen des kennzeichnenden Teils des Patentanspruchs 1 bzw. eine entsprechende gattungsgemäße Vorrichtung mit den Merkmalen des kennzeichnenden Teils des Patentanspruchs 9 gelöst.

Vorteilhafte Ausführungen und Weiterbildungen der Erfindung sind Gegenstand der Unteransprüche.

Die Erfindung umfasst ein Verfahren zur Zentrierdatenermittlung gemäß des unabhängigen Aspruchs 1.

In entsprechender Weise ist Gegenstand der Erfindung auch eine Vorrichtung zur Zentrierdatenermittlung gemäß des Anspruchs 9.

Die oben angegebene Aufgabe wird durch ein derartiges Verfahren bzw. die entsprechende Vorrichtung vollumfänglich gelöst.

Ein erfindungsgemäß verwendbares Verfahren zur Ermittlung der habituellen Kopfhaltung eines Probanden umfasst die Verfahrensschritte gemäß des Anspruchs 3.

Die entsprechende erfindungsgemäß verwendbare Vorrichtung zur Ermittlung der habituellen Kopfhaltung des Probanden umfasst in korrespondierender Weise die Merkmale gemäß des Anspruchs 12.

Durch das vorstehend skizzierte Verfahren bzw. die insbesondere Durchführung dieses Verfahrens ausgebildete entsprechende Vorrichtung wird vermieden, dass die habituelle Kopfhaltung des Probanden irrtümlich als eine zufällig zu einem Zeitpunkt aufgenommene bzw. bestimmte Kopfhaltung angenommen wird, die gerade nicht seiner gewöhnlichen Kopfhaltung entspricht.

Die Annahme der habituellen Kopfhaltung als die aus einer über eine gewisse Zeit hinweg aufgezeichneten Kopfhaltung ermittelte bevorzugte Kopfhaltung ermöglicht es z.B. auch, die bei der Zentriermessung (z.B. mit Hilfe der unter den Bezeichnungen Relaxed Vision Terminal und Video Infral von der Anmelderin vertriebenen Videozentriergeräten) gemessene Kopfhaltung zu korrigieren, so dass die Zentrierdaten auf Basis der "normalen" und nicht der zufälligen Kopfhaltung bestimmt werden.

Bei dem erfindungsgemäß verwendbaren Verfahren zur Ermittlung der habituellen Kopfhaltung eines Probanden kann die Zeitspanne, innerhalb der die Kopfhaltung aufgezeichnet wird, vorgegeben werden. So kann zum Beispiel die Kopfhaltung nur während der Zeit aufgezeichnet werden, während der sich auch verlässliche Daten zur habituellen Haltung des Kopfes des Probanden entnehmen lassen. Die Aufzeichnung erzwungener und/oder unnatürlicher Kopfhaltungen kann vermieden werden. Darüber hinaus lässt sich die Zeitspanne individuell so festlegen, dass das aufgenommene Datenmaterial eine hinreichend verlässliche Aussage über die tatsächliche habituelle Kopfhaltung zulässt. Günstig ist es z.B., wenn aus der Aufzeichnung nur solche Zeitabschnitte zur Ermittlung der habituellen Kopfhaltung ausgewählt werden, innerhalb derer der Proband auch eine Kopf- und/oder Körperhaltung eingenommen hat, die ergonomischen Grundsätzen entspricht.

Als bevorzugte Kopfhaltung ist die Kopfhaltung zu verstehen, in der der Proband seinen Kopf bei einer vorgegebenen Sehaufgabe mit einer gewissen Wahrscheinlichkeit hält. Typische Werte für diese Wahrscheinlichkeit sind z.B. über 30 %, über 50 %, über 80 % oder gar über 90 % für die bestimmte Sehaufgabe. Eine derartige Analyse lässt sich mit üblichen. mathematischen/statistischen Methoden durchführen. Geeignete Software Algorithmen können bei Bedarf automatisch oder mit Unterstützung durch den Benutzer Extremwerte (Ausreißer) aus der aufgezeichneten Datenmenge der habituellen Kopfhaltung eliminieren, bevor eine Bestimmung einer typischen Kopfhaltung erfolgt. Es erfolgt dann z.B. ein Auswahl aus der Menge dieser wahrscheinlichsten Kopfhaltungen.

Als bevorzugte Kopfhaltung kann auch die aufgezeichnete Haltung des Kopfes gewählt werden, in der der Proband den Kopf mit der größten Wahrscheinlichkeit hält.

Obwohl es grundsätzlich möglich ist, dem Probanden freizustellen, in welcher Körperhaltung und/oder was er bei der Aufzeichnung seiner Kopfhaltung betrachtet, ist es vielfach günstig, diesem bestimmte Sehaufgaben aufzuerlegen. Insbesondere wenn die Kopfhaltung Eingang in die Brillenglasberechnung oder die Zentrierung eines Brillenglases findet, ist es sinnvoll, besondere Aufmerksamkeit den Blickrichtungen für die Nähe und/oder die Ferne zu widmen. Die Erfindung sieht z.B. vor, als bevorzugte Kopfhaltung eine der aufgezeichneten Haltungen zu wählen, in der der Proband den Kopf beim Blick in die Nähe und/oder beim Blick in die Ferne hält.

Weiterhin ist es sinnvoll, die Körperhaltung des Probanden zu beachten. Beim Blick in die Ferne wird eine Person eher stehen, wohingegen eine Person beim Blick in die Nähe, wie z.B. beim Lesen, bevorzugt eine sitzende Position einnimmt. Die Erfindung sieht daher vor, als bevorzugte Kopfhaltung eine der aufgezeichneten Haltungen zu wählen, während der der Proband steht und/oder sitzt.

Die habituelle Kopfhaltung hängt in besonderem Maß von der Tätigkeit des Probanden und den visuellen Eindrücken, die der Proband wahrnimmt ab. Es ist daher weiter günstig, dem Probanden konkrete Alltagssituationen und/oder alltägliche Sehaufgaben darzubieten unter denen seine habituelle Kopfhaltung bestimmt werden soll. Der hiermit verbundene Vorteil liegt darin, dass der Proband/Kunde von der eigentlichen Messsituation abgelenkt ist und damit keine unnatürliche Kopfhaltung einnimmt. Die Sehaufgaben können darin bestehen, dass der Proband/Kunde sich z.B. im Laden des Optikers, der die habituelle Kopfhaltung aufzeichnet frei bewegt und z.B. die Auslagen betrachtet oder dass er z.B. Verkehrssituationen verfolgt, die über ein elektronisches Medium, wie ein Display, einen Beamer usw., dargestellt werden, wie dies z.B. in der US 2007/0229761 A1 beschrieben ist oder dass er gebeten wird ein Schriftstück durchzulesen. Die Sehaufgaben werden z.B. so gewählt, dass eine typische Kopfhaltung im Stehen und/oder im Sitzen bestimmt werden kann. Bei der Zentrierdatenermittlung für eine Lesebrille wird z.B. die habituelle Kopfhaltung zu berücksichtigen sein, während der der Proband sitzt und liest.

Konkret können dem Probanden/Kunden zur dynamischen Ermittlung der habituellen Kopfhaltung über ein Zeitintervall Alltagssituationen bzw. alltägliche Sehaufgaben im Nah- und/oder Fernbereich dargeboten werden. In dieser Zeit werden die Kopfhaltung, ggf. in Kombination mit der zugehörigen Körperhaltung und ggf. auch die (relative) Position wie z.B. die Neigung der Brille bzw. der Brillengläser, die der Proband trägt, fortlaufend gemessen. Die Messung erfolgt so, dass der Proband/Kunde davon nicht beeinträchtigt wird. Im Anschluss an die Messung kann die jeweils häufigste Kopfhaltung, soweit mit aufgezeichnet auch die zugehörige Körperhaltung- und ggf. auch die Position, insbesondere Neigung der Brille bzw. der Brillengläser bestimmt und später bei der Ermittlung der Zentrierdaten berücksichtigt werden. Insbesondere können die Durchblickpunkte bei Nah- und/oder Fernsehaufgaben, der sogenannte Inset und/oder die Korridorlänge bestimmt werden. Konkret kann z.B. aus der Kopfhaltung beim Sehen im Nah- und Fernbereich bei bekannter Position des Sehziels der Durchblickpunkt durch das Brillenglas bestimmt werden. Sind die Durchblickpunkte bekannt, kann damit auch die Größe des Insets bestimmt werden, sowie die Länge des Korridors des Nahteils. Ein eventueller Unterschied zwischen Kopfrichtung und Blickrichtung kann bei der Bestimmung der Durchblickpunkte und/oder des Insets mit berücksichtigt werden.

Die Kopfhaltung des Probanden kann auf unterschiedlichste Art und Weise ermittelt werden. So kann z.B. die Aufzeichnung der Kopfhaltung mittels eines am Kopf des Probanden angebrachten Positionsaufzeichnungsgeräts durchgeführt werden. Dieses Positionsaufzeichnungsgerät kann mittelbar also z.B. am Brillenfassungsbügel oder unmittelbar am Kopf des Klienten befestigt sein. Dieses Positionsaufzeichnungsgerät kann nach dem Prinzip eines Datenloggers mit anschließendem Datentransfer über eine Basisstation funktionieren oder der Datentransfer kann zeitgleich über eine drahtlose Verbindung, wie WPAN (Wireless Personal Area Network), nämlich z.B. via Bluetooth, oder WLAN (Wireless Local Area Network) online während des gesamten Messintervalls erfolgen. Das Positionsaufzeichnungsgerät kann entweder absolute Lageinformationen messen oder aber Änderungen in der Lage. Im zweiten Fall muss der Messwertaufnehmer vor und/oder nach dem Messvorgang in eine Referenzlage gebracht werden, deren Ausrichtung bekannt ist. Als Referenzlage kann z.B. eine horizontale Tischfläche oder die Auflage einer speziell dafür vorgesehenen Halterung dienen.

Die Aufzeichnung der Kopfhaltung kann zusätzlich oder alternativ mittels eines Bildaufzeichnungsgeräts durchgeführt werden. Ein derartiges Bildaufzeichnungsgerät kann z.B. ein Video- oder Digitalkamerasystem mit angeschlossener automatisierter Bildauswertung sein. Die Bilddaten können entweder als zweidimensionale (2D) Daten direkt oder nach Erstellung eines dreidimensionalen (3D) Modells als 3D-Daten weiterverarbeitet werden. Aus diesen Daten können z.B. auch typische Parameter wie Vorneigung, Seitenneigung, usw., der Brille bestimmt werden.

Bei dem erfindungsgemäßen Verfahren zur Zentrierdatenermittlung wird die bestimmte tatsächliche momentane Kopfhaltung mit Hilfe der bestimmten habituellen Kopfhaltung derart korrigiert, dass die Zentrierdaten auf Basis der habituellen und nicht der zufälligen Kopfhaltung bestimmt werden. Die Korrektur erfolgt indem die Abweichung der momentanen Kopfhaltung von der habituellen Kopfhaltung ermittelt wird und anhand dieser Abweichung eine Umrechnung/Korrektur der Zentrierwerte erfolgt.

Die ermittelte habituelle Kopfhaltung des Probanden/Kunden und der ggf. ebenfalls ermittelte Vorneigungswinkel der Brille können auch zur erzwungenen Ausrichtung des Kunden für die konventionelle Zentrierdatenermittlung dienen, indem bei Erreichen des angestrebten Vomeigungswinkels der Brille eine automatische Auslösung der bildgebenden Einheiten eingeleitet wird bzw. ein Signal dem Operator gegeben wird, dass nun die Zentrierdatenermittlung erfolgen kann.

Die Erfindung sieht insbesondere auch vor, dass die vorstehend beschriebenen Verfahren zur Ermittlung der habituellen Kopfhaltung sowie zur Zentrierdatenermittlung in Form von in einem Computer ausführbaren Computerprogrammen mit Programmcodes vorliegen. Die Computerprogramme können auf einem maschinenlesbaren Datenträger gespeichert sein.

Der Vollständigkeit halber wird darauf hingewiesen, dass die o.a. Aufzeichnungseinrichtung zur Aufzeichnung der Kopfhaltung über eine Zeitspanne ein am Kopf des Probanden anbringbares Positionsaufzeichnungsgerät und/oder ein Bildaufzeichnungsgerät umfassen kann. Positionsaufzeichnungsgerät und/oder Bildaufzeichnungsgerät können z.B. in der vorstehend beschriebenen Weise ausgebildet sein. Die Ermittlungseinrichtung zur Ermittlung einer bevorzugten Kopfhaltung aus der Aufzeichnung kann einen Computer umfassen.

Die Kopfhaltungsbestimmungseinrichtung zur Bestimmung der tatsächlichen momentanen Kopfhaltung eines Probanden kann ein Positionsaufzeichnungsgerät und/oder ein Bildaufzeichnungsgerät, insbesondere der vorstehend beschriebenen Art, umfassen.

Die Ermittlungseinrichtung zur Ermittlung der Anordnung einer Brillenfassung relativ zur bestimmten tatsächlichen momentanen Kopfhaltung kann ein Bildaufzeichnungsgerät, insbesondere eine Video- oder Digitalkamera, und/oder einen Computer umfassen.

Die Erfindung wird nunmehr anhand der Zeichnung näher beschrieben. Es zeigen:
- Figur 1: eine schematische Darstellung einer erfindungsgemäßen Vorrichtung zur Zentrierdatenermittlung mit erfindungsgemäßer Vorrichtung zur Ermittlung der habituellen Kopfhaltung eines Probanden;
- Figur 2: Aufnahmen des Gesichts eines Kunden mit angepasster Fassung in Nullblickrichtung
a. von vorn
b. von der Seite
c. von vorn mit Zentrierdaten, nämlich x, y
d. von der Seite mit Zentrierdaten, nämlich Hornhautscheitelabstand und Fassungsvorneigung

Es wird angenommen, dass ein Kunde ein Optikergeschäft zum Zwecke des Erwerbs einer neuen Brille aufsucht. Zunächst wird er hinsichtlich der für ihn in Frage kommenden Brillengläser und Fassungen beraten. Nach erfolgter Auswahl der Fassung erfolgt die eigentliche Zentrierdatenermittlung mit Hilfe einer erfindungsgemäßen Vorrichtung.

Die Figur 1 zeigt eine erfindungsgemäße Vorrichtung zur Zentrierdatenermittlung 100. Diese Vorrichtung zur Zentrierdatenermittlung 100 umfasst ein Videozentriergerät 110 mit Videokamera 112 und Computer 114 mit Tastatur 116, Bildschirm 118 und Speichermedium 120. Es handelt sich um ein kalibriertes System, also ein System zur Aufnahme und Bestimmung von Abmessungen.

Das Gesicht des Kunden wird mit angepasster Fassung 202 in Nullblickrichtung von vorn und von der Seite mit der Videokamera 112 aufgenommen, wie dies in den Figuren 2a) und b) dargestellt ist.

Mit der auf den Bildschirm 118 dargestellten Frontaufnahme (Figur 2c) wird zunächst die Fassungsform 202 erfasst. Weiterhin werden die Pupillenmitten 204, z.B. im Koinzidenzverfahren, relativ zur Fassungsform 202 ermittelt. Mit der Seitenaufnahme werden der Hornhautscheitelabstand HSA und die Fassungsvorneigung ϕ zur Erfüllung der Augendrehpunktforderung ermittelt, wie dies in Figur 2d) dargestellt ist.

Erfindungsgemäß ist nunmehr vorgesehen, die habituelle Kopfhaltung und die Neigung der Brille z.B. mit Hilfe der kalibrierten Videokamera über einen längeren Zeitraum aufzuzeichnen und hieraus eine habituelle Kopfhaltung und eine bevorzugte Neigung der Brille zu ermitteln.

Die somit ermittelte Kopfhaltung und der damit zusammenhängende Vorneigungswinkel der Brille sowie andere Messdaten wie z.B. die Anordnung der Brille in der Fassungs- oder Scheibenebene in x, y dienen zur Umrechnung der mittels des Videozentriergeräts 110 konventionell ermittelten Zentrierdaten (z.B. Fernbezugspunkt 206, Nahmesskreis 208), falls bei deren Bestimmung der Kunde eine von der normalen Kopfhaltung abweichende Kopfhaltung eingenommen hatte (vgl. Figur 2c).

## Patentansprüche (Patentansprüche für folgende(n) Vertragsstaat(en): AL,AT,BE,BG,CH,CY,CZ,DK,EE,ES,FI,FR,GB,GR,HR,HU,IE,IS,IT,LT,LU,LV,MC,MK,MT,NL,NO,PL,PT,RO,SE,SI,SK,SM,TR)

1. Verfahren zur Zentrierdatenermittlung, bei dem
a. eine tatsächliche momentane Kopfhaltung eines Probanden bestimmt wird,
b. eine Anordnung einer Brillenfassung (202) relativ zur bestimmten tatsächlichen momentanen Kopfhaltung ermittelt wird, wobei
c. mit Hilfe eines automatisierten Verfahrens eine habituelle Kopfhaltung bestimmt wird und
d. die bestimmte tatsächliche momentane Kopfhaltung mit Hilfe der bestimmten habituellen Kopfhaltung korrigiert wird, **dadurch gekennzeichnet, dass** die Zentrierdaten auf Basis der habituellen und nicht der zufälligen tatsächlichen momentanen Kopfhaltung bestimmt werden, indem für die Korrektur eine Abweichung der tatsächlichen momentanen Kopfhaltung von der bestimmten habituellen Kopfhaltung ermittelt wird und anhand dieser Abweichung eine Umrechnung der Zentrierwerte erfolgt.

2. Verfahren nach Anspruch 1, **dadurch gekennzeichnet, dass** in Schritt c. die Neigung (ϕ) der Brillenfassung (202) mit bestimmt wird.

3. Verfahren nach einem der Ansprüche 1 oder 2, **dadurch gekennzeichnet, dass** die habituelle Kopfhaltung mit einem Verfahren mit folgenden Schritten bestimmt wird:
a. Aufzeichnung der Kopfhaltung in einer Messsituation über eine Zeitspanne wenn der Proband von der Messsituation abgelenkt ist,
b. Ermittlung einer bevorzugten Kopfhaltung aus der Aufzeichnung.

4. Verfahren nach Anspruch 3, **dadurch gekennzeichnet, dass** die Zeitspanne vorgegeben wird.

5. Verfahren nach einem der Ansprüche 3 oder 4, **dadurch gekennzeichnet, dass** als bevorzugte Kopfhaltung die aufgezeichnete Haltung gewählt wird, in der der Proband den Kopf bei einer vorgegebenen Sehaufgabe mit der größten Wahrscheinlichkeit hält.

6. Verfahren nach einem der Ansprüche 3 bis 5, **dadurch gekennzeichnet, dass** als bevorzugte Kopfhaltung eine der aufgezeichneten Haltungen gewählt wird, in der der Proband den Kopf beim Blick in die Nähe und/oder beim Blick in die Ferne hält und/oder dass als bevorzugte Kopfhaltung eine der aufgezeichneten Haltungen gewählt wird, während der der Proband steht und/oder sitzt.

7. Verfahren nach einem der Ansprüche 3 bis 6, **dadurch gekennzeichnet, dass** dem Probanden Alltagssituationen und/oder alltägliche Sehaufgaben dargeboten werden.

8. Verfahren nach einem der Ansprüche 3 bis 7, **dadurch gekennzeichnet, dass** die Aufzeichnung der Kopfhaltung mittels eines am Kopf des Probanden angebrachten Positionsaufzeichnungsgeräts durchgeführt wird und/oder dass die Aufzeichnung der Kopfhaltung mittels eines Bildaufzeichnungsgeräts (110) durchgeführt wird.

9. Verfahren nach einem der vorangegangenen Ansprüche, **dadurch gekennzeichnet, dass** die bestimmte tatsächliche momentane Kopfhaltung mit Hilfe der bestimmten habituellen Kopfhaltung derart korrigiert wird, dass die Zentrierdaten (ϕ, x, y) auf Basis der habituellen und nicht der zufälligen tatsächlichen momentanen Kopfhaltung bestimmt werden.

10. Verfahren nach Anspruch 9, **dadurch gekennzeichnet, dass** die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.

11. Vorrichtung (100) zur Zentrierdatenermittlung mit
a. einer Kopfhaltungsbestimmungseinrichtung (110), um eine tatsächliche momentane Kopfhaltung eines Probanden zu bestimmen,
b. einer Ermittlungseinrichtung (114), um eine Anordnung einer Brillenfassung relativ zur bestimmten tatsächlichen momentanen Kopfhaltung zu ermitteln,
c. einer Vorrichtung (110) zur automatischen Bestimmung der habituellen Kopfhaltung und
d. einer Korrektureinrichtung (114) zur Korrektur der bestimmten tatsächlichen momentanen Kopfhaltung mit Hilfe der bestimmten habituellen Kopfhaltung, **dadurch gekennzeichnet, dass** die Zentrierdaten auf Basis der habituellen und nicht der zufälligen tatsächlichen momentanen Kopfhaltung bestimmt werden, indem für die Korrektur eine Abweichung der tatsächlichen momentanen Kopfhaltung von der bestimmten habituellen Kopfhaltung ermittelt wird und anhand dieser Abweichung eine Umrechnung der Zentrierwerte erfolgt.

12. Vorrichtung nach Anspruch 11, **dadurch gekennzeichnet, dass** die Kopfhaltungsbestimmungseinrichtung ein Positionsaufzeichnungsgerät und/oder ein Bildaufzeichnungsgerät (112) umfasst.

13. Vorrichtung nach einem der Ansprüche 11 oder 12, **dadurch gekennzeichnet, dass** die Ermittlungseinrichtung ein Bildaufzeichnungsgerät (112) und/oder einen Computer (114) umfasst.

14. Vorrichtung nach einem der Ansprüche 11 bis 13, **dadurch gekennzeichnet, dass** die Vorrichtung zur Bestimmung der habituellen Kopfhaltung mit
a. Eine Aufzeichnungseinrichtung (110) zur Aufzeichnung der Kopfhaltung in einer Messsituation über eine Zeitspanne wenn der Proband von der Messsituation abgelenkt ist und
b. eine Ermittlungseinrichtung (114) zur Ermittlung einer bevorzugten Kopfhaltung aus der Aufzeichnung
umfasst.

15. Vorrichtung nach Anspruch 14, **dadurch gekennzeichnet, dass** die Aufzeichnungseinrichtung (110) ein am Kopf des Probanden anbringbares Positionsaufzeichnungsgerät und/oder ein Bildaufzeichnungsgerät (112) umfasst.

16. Vorrichtung nach einem der Ansprüche 14 oder 15, **dadurch gekennzeichnet, dass** die Ermittlungseinrichtung einen Computer (114) umfasst.

17. Computerprogramm mit Programmcode zur Durchführung des Verfahrens nach einem der Ansprüche 1 bis 10, wenn das Programm in einem Computer (114) einer Vorrichtung (100) nach einem der Ansprüche 11 bis 16 ausgeführt wird.

18. Computerprogramm nach Anspruch 17, gespeichert auf einem maschinenlesbaren Datenträger (120).

## Patentansprüche (Patentansprüche für folgende(n) Vertragsstaat(en): DE)

1. Verfahren zur Zentrierdatenermittlung, bei dem
a. eine tatsächliche momentane Kopfhaltung eines Probanden bestimmt wird,
b. eine Anordnung einer Brillenfassung (202) relativ zur bestimmten tatsächlichen momentanen Kopfhaltung ermittelt wird,
c. mit Hilfe eines automatisierten Verfahrens eine habituelle Kopfhaltung bestimmt wird und
d. die bestimmte tatsächliche momentane Kopfhaltung mit Hilfe der bestimmten habituellen Kopfhaltung korrigiert wird, wobei die bestimmte tatsächliche momentane Kopfhaltung mit Hilfe der bestimmten habituellen Kopfhaltung derart korrigiert, wird, dass die Zentrierdaten (ϕ, x, y) auf Basis der habituellen und nicht der zufälligen Kopfhaltung bestimmt werden, **dadurch gekennzeichnet, dass** die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.

2. Verfahren nach Anspruch 1, **dadurch gekennzeichnet, dass** in Schritt c. die Neigung (ϕ) der Brillenfassung (202) mit bestimmt wird.

3. Verfahren nach einem der Ansprüche 1 oder 2, **dadurch gekennzeichnet, dass** die habituelle Kopfhaltung mit einem Verfahren mit folgenden Schritten bestimmt wird:
a. Aufzeichnung der Kopfhaltung in einer Messsituation über eine Zeitspanne, wenn der Proband von der Messsituation abgelenkt ist,
b. Ermittlung einer bevorzugten Kopfhaltung aus der Aufzeichnung.

4. Verfahren nach Anspruch 3, **dadurch gekennzeichnet, dass** die Zeitspanne vorgegeben wird.

5. Verfahren nach einem der Ansprüche 3 oder 4, **dadurch gekennzeichnet, dass** als bevorzugte Kopfhaltung die aufgezeichnete Haltung gewählt wird, in der der Proband den Kopf bei einer vorgegebenen Sehaufgabe mit der größten Wahrscheinlichkeit hält.

6. Verfahren nach einem der Ansprüche 3 bis 5, **dadurch gekennzeichnet, dass** als bevorzugte Kopfhaltung eine der aufgezeichneten Haltungen gewählt wird, in der der Proband den Kopf beim Blick in die Nähe und/oder beim Blick in die Ferne hält und/oder dass als bevorzugte Kopfhaltung eine der aufgezeichneten Haltungen gewählt wird, während der der Proband steht und/oder sitzt.

7. Verfahren nach einem der Ansprüche 3 bis 6, **dadurch gekennzeichnet, dass** dem Probanden Alltagssituationen und/oder alltägliche Sehaufgaben dargeboten werden.

8. Verfahren nach einem der Ansprüche 3 bis 7, **dadurch gekennzeichnet, dass** die Aufzeichnung der Kopfhaltung mittels eines am Kopf des Probanden angebrachten Positionsaufzeichnungsgeräts durchgeführt wird und/oder dass die Aufzeichnung der Kopfhaltung mittels eines Bildaufzeichnungsgeräts (110) durchgeführt wird.

9. Vorrichtung (100) zur Zentrierdatenermittlung mit
a. einer Kopfhaltungsbestimmungseinrichtung (110), um eine tatsächliche momentane Kopfhaltung eines Probanden zu bestimmen,
b. einer Ermittlungseinrichtung (114), um eine Anordnung einer Brillenfassung relativ zur bestimmten tatsächlichen momentanen Kopfhaltung zu ermitteln,
c. einer Vorrichtung (110) zur automatischen Bestimmung der habituellen Kopfhaltung und
d. einer Korrektureinrichtung (114) zur Korrektur der bestimmten tatsächlichen momentanen Kopfhaltung mit Hilfe der bestimmten habituellen Kopfhaltung vorgesehen sind, wobei die bestimmte tatsächliche momentane Kopfhaltung mit Hilfe der bestimmten habituellen Kopfhaltung derart korrigiert, wird, dass die Zentrierdaten (ϕ, HSA, x, y) auf Basis der habituellen und nicht der zufälligen Kopfhaltung bestimmt werden, **dadurch gekennzeichnet, dass** die bestimmte tatsächliche momentane Kopfhaltung mit Hilfe der bestimmten habituellen Kopfhaltung korrigiert wird, indem die Abweichung der momentanen Kopfhaltung zur habituellen Kopfhaltung ermittelt wird und anhand dieser eine Umrechnung der Zentrierwerte erfolgt.

10. Vorrichtung nach Anspruch 9, **dadurch gekennzeichnet, dass** die Kopfhaltungsbestimmungseinrichtung ein Positionsaufzeichnungsgerät und/oder ein Bildaufzeichnungsgerät (112) umfasst.

11. Vorrichtung nach einem der Ansprüche 9 oder 10, **dadurch gekennzeichnet, dass** die Ermittlungseinrichtung ein Bildaufzeichnungsgerät (112) und/oder einen Computer (114) umfasst.

12. Vorrichtung nach einem der Ansprüche 9 bis 11, **dadurch gekennzeichnet, dass** die Vorrichtung zur Bestimmung der habituellen Kopfhaltung
a. eine Aufzeichnungseinrichtung (110) zur Aufzeichnung der Kopfhaltung in einer Messsituation über eine Zeitspanne, wenn der Proband von der Messsituation abgelenkt ist; und
b. eine Ermittlungseinrichtung (114) zur Ermittlung einer bevorzugten Kopfhaltung aus der Aufzeichnung
umfasst.

13. Vorrichtung nach Anspruch 12, **dadurch gekennzeichnet, dass** die Aufzeichnungseinrichtung (110) ein am Kopf des Probanden anbringbares Positionsaufzeichnungsgerät und/oder ein Bildaufzeichnungsgerät (112) umfasst.

14. Vorrichtung nach einem der Ansprüche 12 oder 13, **dadurch gekennzeichnet, dass** die Ermittlungseinrichtung einen Computer (114) umfasst.

15. Computerprogramm mit Programmcode zur Durchführung des Verfahrens nach einem der Ansprüche 1 bis 8, wenn das Programm in einem Computer (114) einer Vorrichtung (110) nach einem der Ansprüche 9 bis 14 ausgeführt wird.

16. Computerprogramm nach Anspruch 15, gespeichert auf einem maschinenlesbaren Datenträger (120).

## Claims (Claims for the following Contracting State(s): AL,AT,BE,BG,CH,CY,CZ,DK,EE,ES,FI,FR,GB,GR,HR,HU,IE,IS,IT,LT,LU,LV,MC,MK,MT,NL,NO,PL,PT,RO,SE,SI,SK,SM,TR)

1. Method for determining centering data, wherein
a. an actual instantaneous head posture of a subject is determined,
b. an arrangement of a spectacle frame (202) relative to the actual instantaneous head posture determined is determined, wherein
c. a habitual head posture is determined with the aid of an automated method, and
d. the actual instantaneous head posture determined is corrected with the aid of the habitual head posture determined, **characterized in that** the centering data are determined on the basis of the habitual rather than the random actual instantaneous head posture by, for the correction, determining a deviation of the actual instantaneous head posture from the habitual head posture determined and converting the centering values on the basis of this deviation.

2. Method according to Claim 1, **characterized in that** in step c. the inclination (ϕ) of the spectacle frame (202) is concomitantly determined.

3. Method according to either of Claims 1 and 2, **characterized in that** the habitual head posture is determined using a method having the following steps:
a. recording the head posture in a measurement situation over a period of time, when the subject is diverted from the measurement situation,
b. determining a preferred head posture from the recording.

4. Method according to Claim 3, **characterized in that** the time period is predetermined.

5. Method according to either of Claims 3 and 4, **characterized in that** the preferred head posture chosen is the recorded posture in which the subject keeps their head during a predetermined vision task with the highest probability.

6. Method according to any of Claims 3 to 5, **characterized in that** the preferred head posture chosen is one of the recorded postures in which the subject keeps their head during near-range viewing and/or during far-range viewing and/or **in that** the preferred head posture chosen is one of the recorded postures during which the subject is standing and/or sitting.

7. Method according to any of Claims 3 to 6, **characterized in that** the subject is presented with everyday situations and/or everyday vision tasks.

8. Method according to any of Claims 3 to 7, **characterized in that** the recording of the head posture is carried out by means of a position recording device fitted to the subject's head and/or **in that** the recording of the head posture is carried out by means of an image recording device (110).

9. Method according to any of the preceding claims, **characterized in that** the actual instantaneous head posture determined is corrected with the aid of the habitual head posture determined in such a way that the centering data (ϕ, x, y) are determined on the basis of the habitual rather than the random actual instantaneous head posture.

10. Method according to Claim 9, **characterized in that** the actual instantaneous head posture determined is corrected with the aid of the habitual head posture determined by determining the deviation of the instantaneous head posture from the habitual head posture and converting the centering values on the basis of this deviation.

11. Apparatus (100) for determining centering data comprising
a. a head posture determining device (110) for determining an actual instantaneous head posture of a subject,
b. a determining device (114) for determining an arrangement of a spectacle frame relative to the actual instantaneous head posture determined,
c. an apparatus (110) for automatically determining the habitual head posture, and
d. a correction device (114) for correcting the actual instantaneous head posture determined with the aid of the habitual head posture determined, **characterized in that** the centering data are determined on the basis of the habitual rather than the random actual instantaneous head posture by, for the correction, determining a deviation of the actual instantaneous head posture from the habitual head posture determined and converting the centering values on the basis of this deviation.

12. Apparatus according to Claim 11, **characterized in that** the head posture determining device comprises a position recording device and/or an image recording device (112).

13. Apparatus according to either of Claims 11 and 12, **characterized in that** the determining device comprises an image recording device (112) and/or a computer.

14. Apparatus according to any of Claims 11 to 13, **characterized in that** the apparatus for determining the habitual head posture, comprises
a. a recording device (110) for recording the head posture in a measurement situation over a period of time when the subject is diverted from the measurement situation and
b. a determining device (114) for determining a preferred head posture from the recording.

15. Apparatus according to Claim 14, **characterized in that** the recording device (110) comprises a position recording device and/or an image recording device (112) which can be fitted to the subject's head.

16. Apparatus according to either of Claims 14 and 15, **characterized in that** the determining device comprises a computer (114).

17. Computer program comprising program code for carrying out the method according to any of Claims 1 to 10 if the program is executed in a computer (114) of an apparatus (110) according to any of Claims 11 to 16.

18. Computer program according to Claim 17, stored on a machine-readable data carrier (120).

## Claims (Claims for the following Contracting State(s): DE)

1. Method for determining centering data, wherein
a. an actual instantaneous head posture of a subject is determined,
b. an arrangement of a spectacle frame (202) relative to the actual instantaneous head posture determined is determined,
c. a habitual head posture is determined with the aid of an automated method, and
d. the actual instantaneous head posture determined is corrected with the aid of the habitual head posture determined, wherein the actual instantaneous head posture determined is corrected with the aid of the habitual head posture determined in such a way that the centering data (ϕ, x, y) are determined on the basis of the habitual rather than the random head posture, **characterized in that** the actual instantaneous head posture determined is corrected with the aid of the habitual head posture determined by determining the deviation of the instantaneous head posture from the habitual head posture and converting the centering values on the basis of this deviation.

2. Method according to Claim 1, **characterized in that** in step c. the inclination (ϕ) of the spectacle frame (202) is concomitantly determined.

3. Method according to either of Claims 1 and 2, **characterized in that** the habitual head posture is determined using a method having the following steps:
a. recording the head posture in a measurement situation over a period of time, when the subject is diverted from the measurement situation,
b. determining a preferred head posture from the recording.

4. Method according to Claim 3, **characterized in that** the time period is predetermined.

5. Method according to either of Claims 3 and 4, **characterized in that** the preferred head posture chosen is the recorded posture in which the subject keeps their head during a predetermined vision task with the highest probability.

6. Method according to any of Claims 3 to 5, **characterized in that** the preferred head posture chosen is one of the recorded postures in which the subject keeps their head during near-range viewing and/or during far-range viewing and/or **in that** the preferred head posture chosen is one of the recorded postures during which the subject is standing and/or sitting.

7. Method according to any of Claims 3 to 6, **characterized in that** the subject is presented with everyday situations and/or everyday vision tasks.

8. Method according to any of Claims 3 to 7, **characterized in that** the recording of the head posture is carried out by means of a position recording device fitted to the subject's head and/or **in that** the recording of the head posture is carried out by means of an image recording device (110).

9. Apparatus (100) for determining centering data comprising
a. a head posture determining device (110) for determining an actual instantaneous head posture of a subject,
b. a determining device (114) for determining an arrangement of a spectacle frame relative to the actual instantaneous head posture determined,
c. an apparatus (110) for automatically determining the habitual head posture, and
d. a correction device (114) for correcting the actual instantaneous head posture determined with the aid of the habitual head posture determined are provided, wherein the actual instantaneous head posture determined is corrected with the aid of the habitual head posture determined in such a way that the centering data (ϕ, HSA, x, y) are determined on the basis of the habitual rather than the random head posture, **characterized in that** the actual instantaneous head posture determined is corrected with the aid of the habitual head posture determined by determining the deviation of the instantaneous head posture from the habitual head posture and converting the centering values on the basis of this deviation.

10. Apparatus according to Claim 9, **characterized in that** the head posture determining device comprises a position recording device and/or an image recording device (112).

11. Apparatus according to either of Claims 9 and 10, **characterized in that** the determining device comprises an image recording device (112) and/or a computer (114) .

12. Apparatus according to any of Claims 9 to 11, **characterized in that** the apparatus for determining the habitual head posture comprises
a. a recording device (110) for recording the head posture in a measurement situation over a period of time, when the subject is diverted from the measurement situation; and
b. a determining device (114) for determining a preferred head posture from the recording.

13. Apparatus according to Claim 12, **characterized in that** the recording device (110) comprises a position recording device and/or an image recording device (112) which can be fitted to the subject's head.

14. Apparatus according to either of Claims 12 and 13, **characterized in that** the determining device comprises a computer (114).

15. Computer program comprising program code for carrying out the method according to any of Claims 1 to 8 if the program is executed in a computer (114) of an apparatus (110) according to any of Claims 9 to 14.

16. Computer program according to Claim 15, stored on a machine-readable data carrier (120).

## Revendications (Revendications pour l'(les) Etat(s) contractant(s) suivant(s): DE)

1. Procédé de détermination de données de centrage, dans lequel
a. un port de tête momentané effectif d'un sujet est déterminé,
b. une disposition d'une monture de lunettes (202) par rapport au port de tête momentané effectif déterminé est déterminée,
c. un port de tête habituel est déterminé à l'aide d'un procédé automatisé et
d. le port de tête momentané effectif déterminé est corrigé à l'aide du port de tête habituel déterminé, le port de tête momentané effectif déterminé étant corrigé à l'aide du port de tête habituel déterminé de telle sorte que les données de centrage (ϕ, x, y) soient déterminées sur la base du port de tête habituel et non pas sur la base du port de tête aléatoire, **caractérisé en ce que** le port de tête momentané effectif déterminé est corrigé à l'aide du port de tête habituel déterminé, en déterminant l'écart du port de tête momentané par rapport au port de tête habituel et en effectuant à l'aide de celui-ci une conversion des valeurs de centrage.

2. Procédé selon la revendication 1, **caractérisé en ce qu'**à l'étape c., l'inclinaison (ϕ) de la monture de lunettes (202) est déterminée en même temps.

3. Procédé selon l'une quelconque des revendications 1 ou 2, **caractérisé en ce que** le port de tête habituel est déterminé avec un procédé comprenant les étapes suivantes :
a. enregistrement du port de tête dans une situation de mesure pendant un intervalle de temps pendant lequel le sujet est distrait de la situation de mesure,
b. détermination d'un port de tête préféré à partir de l'enregistrement.

4. Procédé selon la revendication 3, **caractérisé en ce que** l'intervalle de temps est prédéfini.

5. Procédé selon l'une quelconque des revendications 3 ou 4, **caractérisé en ce que** l'on sélectionne en tant que port de tête préféré le port enregistré dans lequel le sujet maintient sa tête avec la plus grande probabilité pour une tâche prédéfinie à observer.

6. Procédé selon l'une quelconque des revendications 3 à 5, **caractérisé en ce que** l'on sélectionne en tant que port de tête préféré l'un des ports enregistrés dans lequel le sujet maintient sa tête en regardant à proximité et/ou en regardant au loin et/ou **en ce que** l'on sélectionne en tant que port de tête préféré l'un des ports enregistrés pendant lequel le sujet est debout et/ou est assis.

7. Procédé selon l'une quelconque des revendications 3 à 6, **caractérisé en ce que** l'on offre au sujet des situations de tous les jours et/ou des tâches de tous les jours à observer.

8. Procédé selon l'une quelconque des revendications 3 à 7, **caractérisé en ce que** l'enregistrement du port de tête est effectué au moyen d'un appareil d'enregistrement de position monté sur la tête du sujet et/ou **en ce que** l'enregistrement du port de tête est effectué au moyen d'un appareil d'enregistrement d'images (110).

9. Dispositif (100) de détermination de données de centrage, comprenant
a. un dispositif de détermination du port de tête (110), afin de déterminer un port de tête momentané effectif d'un sujet,
b. dispositif de détermination (114) afin de déterminer une disposition d'une monture de lunettes par rapport au port de tête momentané effectif déterminé,
c. un dispositif (110) pour déterminer automatiquement le port de tête habituel et
d. un dispositif de correction (114) pour corriger le port de tête momentané effectif déterminé à l'aide du port de tête habituel déterminé,
le port de tête momentanée effectif déterminé étant corrigé à l'aide du port de tête habituel déterminé de telle sorte que les données de centrage (ϕ, HSA, x, y) soient déterminées sur la base du port de tête habituel et non pas sur la base du port de tête aléatoire, **caractérisé en ce que** le port de tête momentané effectif déterminé est corrigé à l'aide du port de tête habituel déterminé, en déterminant l'écart du port de tête momentané par rapport au port de tête habituel et en effectuant à l'aide de celui-ci une conversion des valeurs de centrage.

10. Dispositif selon la revendication 9, **caractérisé en ce que** le dispositif de détermination du port de tête comprend un appareil d'enregistrement de position et/ou un appareil d'enregistrement d'images (112).

11. Dispositif selon l'une quelconque des revendications 9 ou 10, **caractérisé en ce que** le dispositif de détermination comprend un appareil d'enregistrement d'images (112) et/ou un ordinateur (114).

12. Dispositif selon l'une quelconque des revendications 9 à 11, **caractérisé en ce que** le dispositif de détermination du port de tête habituel comprend
a. un dispositif d'enregistrement (110) pour enregistrer le port de tête dans une situation de mesure pendant un intervalle de temps pendant lequel le sujet est distrait de la situation de mesure ; et
b. un dispositif de détermination (114) pour déterminer un port de tête préféré à partir de l'enregistrement.

13. Dispositif selon la revendication 12, **caractérisé en ce que** le dispositif d'enregistrement (110) comprend un appareil d'enregistrement de position et/ou un appareil d'enregistrement d'images (112) pouvant être monté(s) sur la tête du sujet.

14. Dispositif selon l'une quelconque des revendications 12 ou 13, **caractérisé en ce que** le dispositif de détermination comprend un ordinateur (114) .

15. Programme informatique avec un code de programme pour mettre en oeuvre le procédé selon l'une quelconque des revendications 1 à 8, lorsque le programme est exécuté dans un ordinateur (114) d'un dispositif (100) selon l'une quelconque des revendications 9 à 14.

16. Programme informatique selon la revendication 15, mémorisé sur un support de données lisible par machine (120).

## Revendications (Revendications pour l'(les) Etat(s) contractant(s) suivant(s): AL,AT,BE,BG,CH,CY,CZ,DK,EE,ES,FI,FR,GB,GR,HR,HU,IE,IS,IT,LT,LU,LV,MC,MK,MT,NL,NO,PL,PT,RO,SE,SI,SK,SM,TR)

1. Procédé de détermination de données de centrage, dans lequel
a. un port de tête momentané effectif d'un sujet est déterminé,
b. une disposition d'une monture de lunettes (202) par rapport au port de tête momentané effectif déterminé est déterminée,
c. un port de tête habituel est déterminé à l'aide d'un procédé automatisé et
d. le port de tête momentané effectif déterminé est corrigé à l'aide du port de tête habituel déterminé, **caractérisé en ce que** les données de centrage sont déterminées sur la base du port de tête habituel et non pas sur la base du port de tête momentané effectif aléatoire, en déterminant, pour la correction, un écart du port de tête momentané effectif par rapport au port de tête habituel déterminé et en effectuant, à l'aide de cet écart, une conversion des valeurs de centrage.

2. Procédé selon la revendication 1, **caractérisé en ce qu'**à l'étape c., l'inclinaison (ϕ) de la monture de lunettes (202) est déterminée en même temps.

3. Procédé selon l'une quelconque des revendications 1 ou 2, **caractérisé en ce que** le port de tête habituel est déterminé avec un procédé comprenant les étapes suivantes :
a. enregistrement du port de tête dans une situation de mesure pendant un intervalle de temps pendant lequel le sujet est distrait de la situation de mesure,
b. détermination d'un port de tête préféré à partir de l'enregistrement.

4. Procédé selon la revendication 3, **caractérisé en ce que** l'intervalle de temps est prédéfini.

5. Procédé selon l'une quelconque des revendications 3 ou 4, **caractérisé en ce que** l'on sélectionne en tant que port de tête préféré le port enregistré dans lequel le sujet maintient sa tête avec la plus grande probabilité pour une tâche prédéfinie à observer.

6. Procédé selon l'une quelconque des revendications 3 à 5, **caractérisé en ce que** l'on sélectionne en tant que port de tête préféré l'un des ports enregistrés dans lequel le sujet maintient sa tête en regardant à proximité et/ou en regardant au loin et/ou **en ce que** l'on sélectionne en tant que port de tête préféré l'un des ports enregistrés pendant lequel le sujet est debout et/ou est assis.

7. Procédé selon l'une quelconque des revendications 3 à 6, **caractérisé en ce que** l'on offre au sujet des situations de tous les jours et/ou des tâches de tous les jours à observer.

8. Procédé selon l'une quelconque des revendications 3 à 7, **caractérisé en ce que** l'enregistrement du port de tête est effectué au moyen d'un appareil d'enregistrement de position monté sur la tête du sujet et/ou **en ce que** l'enregistrement du port de tête est effectué au moyen d'un appareil d'enregistrement d'images (110).

9. Procédé selon l'une quelconque des revendications précédentes, **caractérisé en ce que** le port de tête momentané effectif déterminé est corrigé à l'aide du port de tête habituel déterminé de telle sorte que les données de centrage (ϕ, x, y) soient déterminées sur la base du port de tête habituel et non pas sur la base du port de tête momentané effectif aléatoire.

10. Procédé selon la revendication 9, **caractérisé en ce que** le port de tête momentané effectif déterminé est corrigé à l'aide du port de tête habituel déterminé, en déterminant l'écart du port de tête momentané par rapport au port de tête habituel et en effectuant à l'aide de celui-ci une conversion des valeurs de centrage.

11. Dispositif (100) de détermination de données de centrage, comprenant
a. un dispositif de détermination du port de tête (110), afin de déterminer un port de tête momentané effectif d'un sujet,
b. dispositif de détermination (114) afin de déterminer une disposition d'une monture de lunettes par rapport au port de tête momentané effectif déterminé,
c. un dispositif (110) pour déterminer automatiquement le port de tête habituel et
d. un dispositif de correction (114) pour corriger le port de tête momentané effectif déterminé à l'aide du port de tête habituel déterminé,
**caractérisé en ce que** les données de centrage sont déterminées sur la base du port de tête habituel et non pas sur la base du port de tête momentané effectif aléatoire, en déterminant pour la correction un écart du port de tête momentané effectif par rapport au port de tête habituel déterminé et en effectuant à l'aide de cet écart une conversion des valeurs de centrage.

12. Dispositif selon la revendication 11, **caractérisé en ce que** le dispositif de détermination du port de tête comprend un appareil d'enregistrement de position et/ou un appareil d'enregistrement d'images (112).

13. Dispositif selon l'une quelconque des revendications 11 ou 12, **caractérisé en ce que** le dispositif de détermination comprend un appareil d'enregistrement d'images (112) et/ou un ordinateur (114).

14. Dispositif selon l'une quelconque des revendications 11 à 13, **caractérisé en ce que** le dispositif de détermination du port de tête habituel comprend
a. un dispositif d'enregistrement (110) pour enregistrer le port de tête dans une situation de mesure pendant un intervalle de temps pendant lequel le sujet est distrait de la situation de mesure et
b. un dispositif de détermination (114) pour déterminer un port de tête préféré à partir de l'enregistrement.

15. Dispositif selon la revendication 14, **caractérisé en ce que** le dispositif d'enregistrement (110) comprend un appareil d'enregistrement de position et/ou un appareil d'enregistrement d'images (112) pouvant être monté(s) sur la tête du sujet.

16. Dispositif selon l'une quelconque des revendications 14 ou 15, **caractérisé en ce que** le dispositif de détermination comprend un ordinateur (114) .

17. Programme informatique avec un code de programme pour mettre en oeuvre le procédé selon l'une quelconque des revendications 1 à 10, lorsque le programme est exécuté dans un ordinateur (114) d'un dispositif (100) selon l'une quelconque des revendications 11 à 16.

18. Programme informatique selon la revendication 17, mémorisé sur un support de données lisible par machine (120).
